# EUROPEAN PATENT APPLICATION

(11) **EP 4 697 350 A1**
(43) Date of publication of application: **18.02.2026**
(21) Application number: 24196879.1
(22) Date of filing: 28.08.2024
(51) Int. Cl.: G16H 40/63, G06T 11/20, G06T 19/00, G16H 50/50, G16H 50/30

(54) **CONTROLLING DISPLAY**

(30) Priority: 14.08.2024 US 202463682946 P
(71) Applicant: Koninklijke Philips N.V., 5656 AG Eindhoven (NL)
(72) Inventor: HUIJBREGTS, Laurentia Johanna, 5656 AG Eindhoven (NL); TESSEL, Uli, 5656 AG Eindhoven (NL); BEAULIEU, Tammy, 5656AG Eindhoven (NL)
(74) Representative: Philips Intellectual Property & Standards

(57) **Abstract**

Proposed concepts thus aim to provide schemes, solutions, concepts, designs, methods and systems pertaining to controlling a display. In particular, embodiments aim to provide a method for controlling a display by generating a display control signal which defines the display of a predicted future avatar of the subject. In this way, for example, a clinician can be provided with a display with which they can quickly assess a future state of the subject (predicted by an algorithm). Not only that, but by using at least one visual parameter of the predicted future avatar (e.g., size or transparency), a clinician can also be provided with the probability of the predicted future status of the subject at a glance.

## Description

### FIELD OF THE INVENTION

This invention relates to the field of controlling a display.

### BACKGROUND OF THE INVENTION

Avatars have recently begun being used on medical displays to provide visual representations of a subject's vital signs (and/or other relevant clinical parameters) through, for example, a virtual model of the monitored subject, indicating the healthiness of their vital signs through color, shape and/or animation. In this way, an avatar can provide an overview of a subject's health state at a quick glance. An avatar typically has a set of parameter thresholds for individual clinical parameters, and the visualization of said clinical parameters typically depends on the configuration of this parameter set. For example, if the measured values exceed or fall below a threshold, an animation can represent this transition accordingly.

Recently, algorithms have been derived to predict a likelihood of a subject status (i.e., one or more clinical parameters) at some point in the future, or in other words, a risk score that the subject will exhibit a given condition within a certain time window. To aid clinicians in making clinical decisions, it would be beneficial if they could be provided with this information in an effective manner.

### SUMMARY OF THE INVENTION

The invention is defined by the claims.

According to examples in accordance with an aspect of the invention, there is provided a computer-implemented method for controlling a display.

The method comprises: generating a display control signal for controlling a display, the display control signal describing a predicted future avatar of the subject; wherein the predicted future avatar visually indicates at least one future clinical parameter of the subject within a first time frame, wherein the at least one future clinical parameter is predicted by a first algorithm; and wherein a probability of the at least one future clinical parameter is indicated by at least one visual parameter of the predicted future avatar.

Proposed concepts thus aim to provide schemes, solutions, concepts, designs, methods and systems pertaining to controlling a display. In particular, embodiments aim to provide a method for controlling a display by generating a display control signal which defines the display of a predicted future avatar of the subject. In this way, for example, a clinician can be provided with a display with which they can quickly assess a future state of the subject (predicted by an algorithm). Not only that, but by using at least one visual parameter of the predicted future avatar (e.g., size or transparency), a clinician can also be provided with the probability of the predicted future status of the subject at a glance.

In other words, it is proposed that an avatar can not only be used to provide a visual representation of a subject's current status but can also/instead be utilized to represent their predicted future status. A display can thus be provided displaying a predicted future avatar of the subject, indicating at least one clinical parameter of the subject. A visual parameter of the predicted future avatar can then also be manipulated to efficiently and effectively indicate the probability of the prediction. In this way, in a single glance, a clinician can be provided with an overview of a subject's future health state, and the probability of said future health state.

For example, this method of controlling a display could be utilized to control a bedside medical display, a central display (e.g., for overseeing a number of subjects in a ward, for example), a VR/AR/MR headset, or any other suitable display, as would be understood by the skilled person.

In summary, the method provides a way to combine a multitude of data into a single display which can effectively convey a large amount of potentially complicated data in a simple manner, such that the information can be easily understood in a single glance, thus allowing a clinician to more efficiently spend their time.

In some embodiments, a visual parameter may comprise at least one of: position on the display; size; brightness; and transparency. These may all provide particularly effective visual parameters for indicating a probability of the future clinical parameter. For example, if the probability is low, the predicted future avatar can be small, and if the probability is high, the predicted future avatar can be large.

In some embodiments, the first time frame may be indicated by a different at least one visual parameter of the predicted future avatar to the at least one visual parameter used to indicate the probability. For example, if the probability is indicated by size, the time frame may be indicated by position on the screen, such that if the time frame is long, the predicted future avatar can be positioned to the far side of the screen, and if the time frame is short, the predicted future avatar can be positioned near the center of the screen.

In some embodiments, the display control signal may further describe a current avatar of the subject, the current avatar visually indicating at least one current clinical parameter of the subject. In this way, a display can be provided displaying both a current avatar of the subject and a predicted future avatar of the subject. In a single glance, a clinician can thus be provided with an overview of a subject's current health state, their future health state, and the probability of said future health state.

In some embodiments, the method may further comprise, if the predicted future avatar matches the current avatar, adjusting the display control signal to generate a modified display control signal describing the non-display of the predicted future avatar. For example, if the subject's clinical status is predicted to stay constant, then there is no need to show a clinician two identical avatars, and this may, in fact, confuse the clinician if they are looking for differences. Therefore, by essentially hiding the predicted future avatar, the clinician can be effectively told that no changes are expected in the future.

In some embodiments, the at least one future clinical parameter may comprise the same clinical parameters as the at least one current clinical parameter. In this way, a clinician may be provided with a more useful comparison between the subject's current state and their predicted future state.

In some embodiments, the method may further comprise, if the probability of the at least one future clinical parameter is less than a predetermined threshold, adjusting the display control signal to generate a modified display control signal describing the non-display of the predicted future avatar. In this way, if the algorithm is not confident about the prediction, it may be hidden so that the clinician may not be provided with likely-misleading information.

In some embodiments, the method may further comprise, if the first time frame is longer than a preset time frame, adjusting the display control signal to generate a modified display control signal describing the non-display of the predicted future avatar. In this way, the clinician may not be provided with information that is not currently relevant due to the prediction being for too far a point in the future.

In some embodiments, the display control signal may further describe a past avatar, wherein the past avatar indicates at least one past clinical parameter of the subject. In this way, a clinician may be provided with a display that combines information about the current state of the subject, the predicted future state of the subject, and the past state of the subject in an easy to quickly assess manner.

In some embodiments, the display control signal may further describe a second predicted future avatar of the subject, wherein the second predicted future avatar may indicate at least one second future clinical parameter of the subject within a second time frame, wherein the at least one second future clinical parameter may be predicted by a second algorithm; and wherein a probability of the at least one second predicted clinical parameter may be indicated by at least one visual parameter of the second predicted future avatar. In this way, a clinician may be effectively provided with additional predictions about the subject.

In some embodiments, a clinical parameter may comprise at least one of: SpO2; heart rate; pulse rate; respiration rate; ST elevation; blood pressure; central venous pressure; end-tidal CO2; body temperature; cardiac output; cardiac index; brain activity; relaxation status; FiO2; airway pressure; tidal volume; hypotension prediction; hemodynamic instability prediction; sepsis prediction; and hypoxia prediction. These may all be useful clinical parameters for a clinician to be aware of.

In some embodiments, the display control signal may further describe a time scale, and wherein the first time frame may be indicated by the predicted future avatar's position along the time scale. This may be a particularly effective way to quickly indicate the first time frame.

In some embodiments, an avatar may comprise a graphical representation of a living being. This may be a particularly effective form of avatar for indicating clinical parameters.

In some embodiments, the living being may comprise a person or an animal. These may be particularly effective forms of avatar for indicating clinical parameters for people and/or animals.

In some embodiments, the second algorithm may be different to the first algorithm. In this way, a clinician may be provided with the predictions of different algorithms, for instance, allowing them to quickly compare and contrast the predictions or appreciate them together.

According to another aspect of the invention, there is provided a computer program comprising code means for implementing the method of any herein disclosed method when said program is run on a processing system.

According to another aspect of the invention, there is provided a system for controlling a display. The system comprising a display; and a processor configured to: generate a display control signal for controlling a display, the display control signal describing a predicted future avatar of the subject; wherein the predicted future avatar visually indicates at least one future clinical parameter of the subject within a first time frame, wherein the at least one future clinical parameter is predicted by a first algorithm; and wherein a probability of the at least one future clinical parameter is indicated by at least one visual parameter of the predicted future avatar.

Thus, there may be proposed concepts for controlling a display, and this may be done based on generating a display control signal which describes a predicted future avatar of a subject, as well as indicating the probability of the predicted future avatar using a visual parameter of the predicted future avatar.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling a display according to a proposed embodiment;
Fig. 2 is a flow diagram of a method for controlling a display according to a proposed embodiment;
Fig. 3 is a simplified block diagram of a system for controlling a display according to a proposed embodiment; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling a display. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling a display. This can be achieved by generating a display control signal which defines the display of a predicted future avatar of the subject. In this way, for example, a clinician can be provided with a display with which they can quickly assess the future state of the subject (predicted by an algorithm). Not only that, but by using at least one visual parameter of the predicted future avatar (e.g., size or transparency), a clinician can also be provided with the probability of the predicted future status of the subject at a glance.

In other words, it is proposed that an avatar can not only be used to provide a visual representation of a subject's current status but can also/instead be utilized to represent their predicted future status. A display can thus be provided displaying a predicted future avatar of the subject, indicating at least one clinical parameter of the subject. A visual parameter of the predicted future avatar can then also be manipulated to efficiently and effectively indicate the probability of the prediction. In this way, in a single glance, a clinician can be provided with an overview of a subject's future health state, and the probability of said future health state.

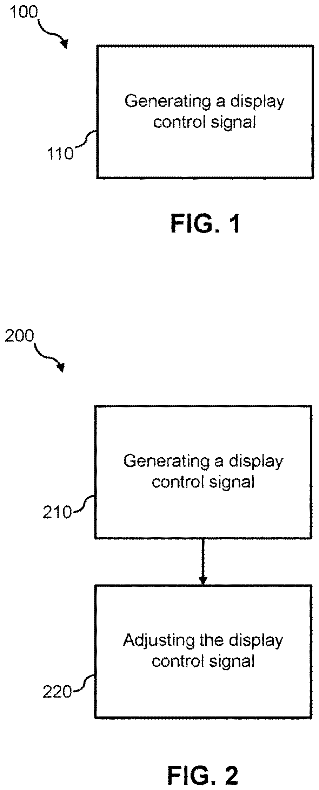

Referring now to Fig. 1, there is depicted a simplified flow diagram of a method 100 for controlling a display according to a proposed embodiment.

The method 100 comprises step 110 of generating a display control signal for controlling a display, the display control signal describing a predicted future avatar of the subject. This step 110 can also be understood as generating a display control signal for controlling a display, the display control signal defining the display of a predicted future avatar of the subject. The display can comprise any suitable display, e.g., a medical display, a monitor, or the display of a VR/AR/MR headset, etc., as the skilled person would understand.

In this embodiment, an avatar comprises a graphical representation of a living being. This is a particularly effective form of avatar for indicating clinical parameters. Specifically in this embodiment, however, the living being comprises a person (i.e., the avatar comprises a graphical representation of a person). This, again, is a particularly effective form of avatar for indicating clinical parameters for people. In other embodiments, however, the living being can be an animal (e.g., for when the subject is an animal, or perhaps for children to pick for themselves).

The predicted future avatar visually indicates at least one future clinical parameter of the subject within a first time frame - for example, the avatar can visually indicate the clinical parameter(s) using at least one of: an icon (e.g., of an organ or of a snowflake to indicate low body temperature, for example); a color (e.g., red to indicate a parameter outside of a normal range); a border (e.g., thick or thin to indicate high or low blood pressure respectively); and an animation (e.g., a heart pumping quickly to indicate a high heart bpm). In embodiments where the display control signal also describes a current avatar of the subject, such as this one, the current avatar visually indicates at least one current clinical parameter of the subject (e.g., in substantially the same way as the current avatar). It should be noted, however, that in other embodiments, the display control signal need not further describe the current avatar of the subject. The predicted future avatar can indicate one or more predicted clinical parameters of the subject within a particular time frame, e.g., within the next twenty-four hours, within the next hour or, for example, in exactly twenty-four hours or between twelve hours and twenty-four hours, etc. The at least one future clinical parameter is predicted by a first algorithm. For example, the first algorithm can be any suitable algorithm for predicting at least one clinical parameter, such as, for example, a linear regression algorithm, a logistic regression algorithm, a decision tree algorithm, a random forest algorithm, a support vector machine (SVM), a k-nearest neighbors (k-NN) algorithm, a naive Bayes algorithm, a neural network, a convolutional neural network (CNN), or a recurrent neural network (RNN), etc. In some embodiments, the first algorithm can comprise multiple algorithms, e.g., each for predicting a different clinical parameter.

It should be noted that in some embodiments, the first algorithm always predicts clinical parameters in the same first time frame, whereas in other embodiments, the first time frame itself can be part of the prediction, as the skilled person would understand. For instance, the first algorithm could predict that a subject will become hypotensive and could also predict that this will happen in three to four hours' time (i.e., this is the first time frame). The first time frame and the probability of said prediction could thus both be indicated by visual parameters of the predicted future avatar.

In this embodiment, a clinical parameter comprises at least one of: SpO2; heart rate; pulse rate; respiration rate; ST elevation; blood pressure; central venous pressure; end-tidal CO2; body temperature; cardiac output; cardiac index; brain activity; relaxation status; FiO2; airway pressure; tidal volume; hypotension prediction; hemodynamic instability prediction; sepsis prediction; and hypoxia prediction. These are all useful clinical parameters for a clinician to be aware of, but of course, in other embodiments, any suitable clinical parameter could be used, as would be understood by the skilled person.

The probability of the at least one future clinical parameter is indicated by at least one visual parameter of the predicted future avatar. For example, the first algorithm which predicts the at least one future clinical parameter will have a probability associated with its prediction (i.e., indicating its confidence in the prediction). This probability can thus be indicated by a visual parameter of the predicted future avatar (i.e., any aspect of the predicted future avatar which can be appreciated/noticed by a human looking at the at the predicted future avatar on the display). For instance, in this embodiment, a visual parameter comprises at least one of: position on the display; distance to the current avatar (of course, this visual parameter only makes sense in embodiments where the display control signal also describes a current avatar); size; brightness; and transparency. These are all particularly effective visual parameters for indicating a probability of the future clinical parameter, though of course, as would be understood by the skilled person, in other embodiments any other suitable visual parameter could be used, such as, for example, a particular animation.

In simple examples, if the probability is low, the predicted future avatar can be small, positioned at the far side of the display, far from the current avatar, dim, and/or nearly fully transparent. Likewise, if the probability is high, the predicted future avatar can be large, positioned at the centre of the display, close to the current avatar, bright, and/or (nearly) fully opaque. In some embodiments, multiple visual parameters can be used to indicate the probability, such as the size and distance from the current avatar being combined, such that low probability is indicated by the predicted future avatar being far from the current avatar and small.

If the avatar indicates two or more clinical parameters and these each have their own probability, then their probabilities can be averaged to reach a value that will then be used for determining the visual parameter (i.e., how bright or big or transparent the avatar is). Of course, as would be appreciated by the skilled person, there other ways of using the multiple probabilities. For instance, the lowest probability could be taken as the value to use for determining the visual parameter, or different parts of the avatar (each relating to one of the clinical parameters) could have different visual parameters to one another. For instance, if a heart rate prediction has a higher probability than the probability for a respiration rate prediction, then a representation of the avatar's heart could be more opaque/bright than a representation of the avatar's lungs.

In some embodiments, the first time frame is also indicated by a different at least one visual parameter of the predicted future avatar to the at least one visual parameter used to indicate the probability. For example, if the probability is indicated by brightness, the time frame can be indicated by size, such that if the time frame is far in the future (e.g., between twenty-two and twenty-four hours away), the predicted future avatar can be relatively small, and if the time frame is closer in the future (e.g., between two and four hours away), the predicted future avatar can be relatively large.

In fact, in this embodiment, the display control signal further describes (i.e., defines the display of) a time scale, and the first time frame is indicated by the predicted future avatar's position along the time scale. This is a particularly effective way to quickly indicate how far in the future the first time frame is. In other embodiments, the display control signal could instead describe a representation of a clock which could then indicate the first time frame. For instance, the clock could show the real-time of the first time frame (e.g., between eight and ten pm) or it could show a number of minutes/hours until the first time frame. Of course, this principle could also be applied to a past avatar, as will now be described.

In this embodiment, the display control signal further describes a past avatar, wherein the past avatar indicates at least one past clinical parameter of the subject. For example, the past clinical parameter could be the value of a clinical parameter of the subject twenty-four hours in the past (i.e., twenty-four hours before the current clinical parameter), or at any other suitable point in time in the past. In this way, a clinician can be provided with a display that combines information about the current state of the subject, the predicted future state of the subject, and the past state of the subject in an easy to quickly assess manner.

For example, the timepoint being represented by the past avatar can also be indicated by at least one visual parameter of the past avatar (e.g., size, distance from the current avatar, transparency, position along a time scale, etc.) For instance, if the past timepoint is relatively far in the past, then the past avatar can be smaller and/or further from the current avatar. It should be noted that as a past avatar is related to real measurements, there is no probability to be indicated.

In this embodiment, the at least one future clinical parameter comprises (at least one of or all) the same clinical parameters as the at least one current clinical parameter (and in embodiments where a past avatar is also present, the at least one past clinical parameter can also comprise (at least one of or all) the same clinical parameters as the at least one future clinical parameter). In this way, a clinician can be provided with a more useful comparison between the subject's current state and their predicted future state.

Referring now to Fig. 2, there is depicted a flow diagram of a method 200 for controlling a display according to a proposed embodiment.

Step 210 is substantially the same as step 110 as described in relation to the method 100 in Fig. 1. However, in step 210, the display control signal further describes a second predicted future avatar of the subject (i.e., in addition to the predicted future avatar and current avatar of the subject), wherein the second predicted future avatar indicates at least one second future clinical parameter of the subject within a second time frame, wherein the at least one second future clinical parameter is predicted by a second algorithm; and wherein a probability of the at least one second predicted clinical parameter is indicated by at least one visual parameter of the second predicted future avatar. In this way, a clinician can be effectively provided with additional predictions about the subject. In this embodiment, the second algorithm is different to the first algorithm, however, this need not be the case. In this way, however, a clinician can be provided with the predictions of different algorithms, for instance, to allow them to quickly compare and contrast the predictions or to appreciate them both simultaneously. It should be noted that the second future clinical parameter can be a different clinical parameter to the first future clinical parameter and that the second time frame can be a different time frame to the first time frame, however, this need not be the case. However, at least one of the second future clinical parameter, the second time frame, and the second algorithm needs to be different to at least one of the first future clinical parameter, the first time frame, and the first algorithm respectively for there to be a purpose to displaying the second predicted future avatar in addition to the first predicted future avatar.

In an example, the two predicted future avatars could vary only by time frame, so that one predicted future avatar could predict a status of the subject in the near future (e.g., within two hours) and the second predicted future avatar could predict a status of the subject in the far future (e.g., between twenty-four hours and forty-eight hours). To indicate this difference in time frame, the first predicted future avatar could be provided nearer the current avatar than the second predicted future avatar. In another example, the first predicted future avatar could indicate predicted arterial blood pressure in twelve hours and the second predicted future avatar could indicate predicted central venous pressure in twelve hours - this could be done by the same or different algorithms.

In step 220, the display control signal is adjusted to generate a modified display control signal describing (i.e. defining) the non-display of the predicted future avatar (i.e., either of the first or the second predicted future avatar). It should be noted that the modified display control signal still describes the display of the other avatars which were described by the non-modified display control signal (i.e., the original display control signal of step 210). In other words, step 220 can be understood as adjusting the display control signal such that the predicted future avatar is hidden on the display.

The display control signal can be adjusted as per step 220 in response to at least one of three conditions: if the predicted future avatar matches the current avatar; if the probability of the at least one future clinical parameter is less than a predetermined threshold; and if the first time frame is longer than a preset time frame. Hiding (i.e., non-displaying) the predicted future avatar if the predicted future avatar matches the current avatar is beneficial because if the subject's status is predicted to stay constant, then there is no need to show a clinician two identical avatars, and this may, in fact, confuse the clinician if they are looking for differences. Therefore, by hiding the predicted future avatar, the clinician is effectively told that no changes are expected in the future. It should also be noted that in some embodiments, if a past avatar matches the current avatar, then the past avatar can also be hidden - in other words, in some embodiments, the past avatar can be shown only if there has been a change within a predetermined time period (and this time period can change depending on the care setting, for example, in the operating room it could be 5/10 minutes, but in the ICU, it could be one hour, and this could be automatically set via location tracking). The past avatar could thus indicate the status of the subject just before the change.

In other words, it mostly only makes sense to show a predictive (or past) avatar if it represents a situation that is different from the current situation. For example, if a subject/patient already has hypotension, then a clinician would not be particularly interested in seeing a prediction that in one hour, the subject is likely to still have hypotension. Instead, it would be more useful, for example, to see that the subject currently has a normal mean arterial pressure, but in an hour is expected to be hypotensive. In fact, in some embodiments, the predicted future avatar can be hidden unless the changes are of a worsening situation (e.g. the blood pressure going from normal to too low), but not when the situation is predicted to get better (e.g. the blood pressure going from too low to normal).

Hiding the predicted future avatar (i.e., adjusting the display control signal so that the predicted future avatar is no longer displayed on the display) if the probability of the at least one future clinical parameter is less than a predetermined threshold (e.g., 50%, 40%, 60%, etc.), facilitates the clinician not being provided with potentially misleading information.

Hiding the predicted future avatar if the first time frame is longer than a preset time frame (e.g., eight hours, twenty-four hours, forty-eight hours, etc.) allows the clinician to not be provided with information that is not currently relevant due to the prediction being for too far a point in the future. This time threshold could also be made dependent on the clinical setting, e.g., in the operating room, the preset time frame could be only 5 minutes, whereas in intensive care, a slightly longer preset time frame of one hour could be more appropriate. In some embodiments, the preset time frame is also adjustable by a user.

It should be noted that the above paragraphs describe hiding the entire predicted future avatar, however, in some embodiments, parts of the predicted future avatar can be hidden independently of one another. For instance, if only one future clinical parameter of the predicted future avatar matches one current clinical parameter of the current clinical avatar (while other clinical parameters of the predicted future and current avatars respectively do not match) then the matching future clinical parameter can be hidden independently of the rest of the predicted future avatar, thus essentially simplifying the predicted future avatar and highlighting only those clinical parameters which are predicted to change. This concept can be similarly applied to clinical parameters being independently hidden if their probability is less than a predetermined threshold or their time frame is longer than a preset time frame.

It should also be noted that in some embodiments, more than two predicted future avatars are generated (not part of the method) but, at most, only two are ever shown. In other words, in some embodiments, the display control signal can exclusively describe two predicted future avatars of the subject (and optionally a current avatar and/or a past avatar of the subject). In this case, all of the predicted future avatars can be hidden except the one or two most probable, soonest, and/or resulting in the worst subject condition. In other words, there can be a prioritization system based on time till occurrence, likelihood, and/or severity, where only the one or two highest priority predicted future avatars are displayed via the display control signal.

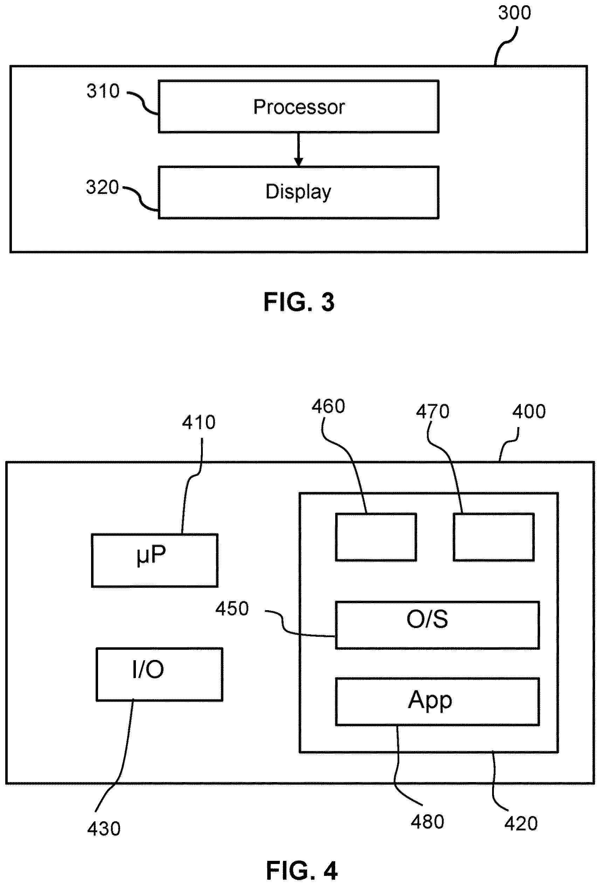

Referring now to Fig. 3, there is depicted a system 300 for controlling a display according to a proposed embodiment. The system 300 comprises a processor 310 and a display 320.

The display 320 can comprise at least one of: a monitor; a virtual reality (VR) headset; an augmented reality (AR) headset; a projector; a television screen; a laptop screen; a tablet display; a smartphone display; an e-ink display; a heads-up display (HUD); a smart mirror; a holographic display; a wearable display, such as smart glasses; an LED matrix display; a flexible OLED display; and any other suitable display, as would be understood by the skilled person.

The processor 310 is configured to: generate a display control signal for controlling a display, the display control signal describing a current avatar of a subject and a predicted future avatar of the subject; wherein the current avatar visually indicates at least one current clinical parameter of the subject; wherein the predicted future avatar visually indicates at least one future clinical parameter of the subject within a first time frame, wherein the at least one future clinical parameter is predicted by a first algorithm; and wherein a probability of the at least one future clinical parameter is indicated by at least one visual parameter of the predicted future avatar.

Fig. 4 illustrates an example of a computer 400 within which one or more parts of an embodiment may be employed. Various operations discussed above may utilize the capabilities of the computer 400. In this regard, it is to be understood that system functional blocks can run on a single computer or may be distributed over several computers and locations (e.g. connected via internet).

The computer 400 includes, but is not limited to, PCs, workstations, laptops, PDAs, palm devices, servers, storages, and the like. Generally, in terms of hardware architecture, the computer 400 may include one or more processors 410, memory 420 and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 460, source code 470, and one or more applications 480 in accordance with exemplary embodiments. As illustrated, the application 480 comprises numerous functional components for implementing the features and operations of the exemplary embodiments. The application 480 of the computer 400 may represent various applications, computational units, logic, functional units, processes, operations, virtual entities, and/or modules in accordance with exemplary embodiments, but the application 480 is not meant to be a limitation.

The operating system 450 controls the execution of other computer programs, and provides scheduling, input-output control, file and data management, memory management, and communication control and related services. It is contemplated by the inventors that the application 480 for implementing exemplary embodiments may be applicable on all commercially available operating systems.

Application 480 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 450. Furthermore, the application 480 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 430 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at start-up, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 480 and the O/S 450 are read, in whole or in part, by the processor 410, perhaps buffered within the processor 410, and then executed.

When the application 480 is implemented in software it should be noted that the application 480 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 480 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-2, and the system of Fig. 3, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for controlling a display, the method comprising:
generating a display control signal (110) for controlling a display, the display control signal describing a predicted future avatar of the subject;
wherein the predicted future avatar visually indicates at least one future clinical parameter of the subject within a first time frame, wherein the at least one future clinical parameter is predicted by a first algorithm; and
wherein a probability of the at least one future clinical parameter is indicated by at least one visual parameter of the predicted future avatar.

2. The computer-implemented method of claim 1, wherein a visual parameter comprises at least one of: position on the display; size; brightness; and transparency.

3. The computer-implemented method of claim 1 or 2, wherein the first time frame is indicated by a different at least one visual parameter of the predicted future avatar to the at least one visual parameter used to indicate the probability.

4. The computer-implemented method of any of claims 1 to 3, wherein the display control signal further describes a current avatar of the subject, the current avatar visually indicating at least one current clinical parameter of the subject.

5. The computer-implemented method of any claim 4, wherein the method further comprises, if the predicted future avatar matches the current avatar, adjusting the display control signal (120) to generate a modified display control signal describing the non-display of the predicted future avatar.

6. The computer-implemented method of claim 4 or 5, wherein the at least one future clinical parameter comprises the same clinical parameters as the at least one current clinical parameter.

7. The computer-implemented method of any of claims 1 to 6, wherein the method further comprises, if the probability of the at least one future clinical parameter is less than a predetermined threshold, adjusting the display control signal (120) to generate a modified display control signal describing the non-display of the predicted future avatar.

8. The computer-implemented method of any of claims 1 to 7, wherein the method further comprises, if the first time frame is longer than a preset time frame, adjusting the display control signal (120) to generate a modified display control signal describing the non-display of the predicted future avatar.

9. The computer-implemented method of any of claims 1 to 8, wherein the display control signal further describes a past avatar, wherein the past avatar indicates at least one past clinical parameter of the subject.

10. The computer-implemented method of any of claims 1 to 9, wherein the display control signal further describes a second predicted future avatar of the subject, wherein the second predicted future avatar indicates at least one second future clinical parameter of the subject within a second time frame, wherein the at least one second future clinical parameter is predicted by a second algorithm; and wherein a probability of the at least one second predicted clinical parameter is indicated by at least one visual parameter of the second predicted future avatar.

11. The computer-implemented method of any of claims 1 to 10, wherein a clinical parameter comprises at least one of: SpO2; heart rate; pulse rate; respiration rate; ST elevation; blood pressure; central venous pressure; end-tidal CO2; body temperature; cardiac output; cardiac index; brain activity; relaxation status; FiO2; airway pressure; tidal volume; hypotension prediction; hemodynamic instability prediction; sepsis prediction; and hypoxia prediction.

12. The computer-implemented method of any of claims 1 to 11, wherein the display control signal further describes a time scale, and wherein the first time frame is indicated by the predicted future avatar's position along the time scale

13. The computer-implemented method of any of claims 1 to 12, wherein an avatar comprises a graphical representation of a living being, and preferably wherein the living being comprises a person or an animal.

14. A computer program product comprising computer program code means which, when executed on a computing device having a processing system, cause the processing system to perform all of the steps of the computer-implemented method according to any of claims 1 to 13.

15. A system (200) for controlling a display, the system comprising:
a display (220); and
a processor (210) configured to:
generate a display control signal for controlling a display, the display control signal describing a predicted future avatar of the subject;
wherein the predicted future avatar visually indicates at least one future clinical parameter of the subject within a first time frame, wherein the at least one future clinical parameter is predicted by a first algorithm; and
wherein a probability of the at least one future clinical parameter is indicated by at least one visual parameter of the predicted future avatar.
